(19) **Europäisches Patentamt**

**European Patent Office**

**Office européen des brevets**

(11) **EP 0 504 459 B1**

(12) **EUROPÄISCHE PATENTSCHRIFT**

(45) Veröffentlichungstag und Bekanntmachung des Hinweises auf die Patenterteilung:
**05.06.1996 Patentblatt 1996/23**

(51) Int. Cl.$^6$: **A61M 11/00**, A61M 15/00

(21) Anmeldenummer: **91104439.4**

(22) Anmeldetag: **21.03.1991**

(54) **Vernebler insbesondere zur Anwendung in Geräten für die Inhalationstherapie**

Nebulizer, in particular for use in inhalation therapy apparatus

Nébuliseur, en particulier à usage dans des appareils pour la thérapie inhalatrice

(84) Benannte Vertragsstaaten:
**DE**

(43) Veröffentlichungstag der Anmeldung:
**23.09.1992 Patentblatt 1992/39**

(73) Patentinhaber: **PAUL RITZAU PARI-WERK GmbH**
**D-82319 Starnberg (DE)**

(72) Erfinder:
• **Knoch, Martin**
**W-8137 Berg (DE)**

• **Brugger, Stephan**
**Starnberg (DE)**

(74) Vertreter: **Hoffmann, Klaus, Dr. rer. nat. et al**
**Hoffmann, Eitle & Partner**
**Patentanwälte**
**Postfach 81 04 20**
**81904 München (DE)**

(56) Entgegenhaltungen:
EP-A- 0 052 284          EP-A- 0 237 507
WO-A-90/15635          FR-A- 2 638 362
US-A- 3 362 405

# Beschreibung

Die Erfindung betrifft einen Vernebler insbesondere zur Anwendung in Geräten für die Inhalationstherapie.

Nicht nur zur Behandlung von Atemwegserkrankungen, sondern in zunehmendem Maße auch zur Verabreichung anderer medizinischer Wirkstoffe wird die Inhalationstherapie angewandt. Bei dieser Therapieart wird der Wirkstoff in Form eines Flüssigkeitsnebels mit sehr kleinen Tröpfchendurchmessern (unter 5 µm) dem Patienten zur Inhalation dargeboten und zusammen mit der Atemluft in die Atemwege transportiert. Für eine sichere Aufnahme des Wirkstoffes ist es erforderlich, daß der Flüssigkeitsnebel und mit ihm der Wirkstoff durch den Atemluftstrom weit in die Atemwege hinein transportiert wird, ohne daß eine vorzeitige Ablagerung im Mund- und Rachenbereich stattfindet. Erst der Transport in die tiefen Atemwege gewährleistet eine effektive Aufnahme des Wirkstoffes über die Oberfläche der Lunge. Ferner ist stets eine zeitliche Koordinierung zwischen dem Inhalationsvorgang und der Erzeugung bzw. Darbietung des Flüssigkeitsnebels erforderlich, mit dem Ziel, während der Einatmungsphase eine ausreichende und gleichzeitig für die Dosierung erfaßbare Menge des Flüssigkeitsnebels bereitzustellen.

Im Hinblick auf diese beiden Anforderungen ist bei der Anwendung von Dosieraerosolen bereits erkannt worden, daß ein mit dem Atemvorgang koordiniert in den Atemluftstrom eingeführter Strahl eines mit einer Zerstäubungsdüse erzeugten Flüssigkeitsnebels zu stark gebündelt in den Mund- und Rachenraum eingeführt wird, als daß der angestrebte Transport in die tiefen Atemwege hinein erreicht wird. Deshalb sind Beruhigungskammern zwischen Zerstäubungsort und Inhalationsort vorgeschlagen worden, etwa in Form eines verlängerten Mundstücks, die zu einer Verteilung und Verlangsamung des Flüssigkeitsnebels führen und eine unkoordinierte Inhalation nach erfolgter Zerstäubung ermöglichen sollen. Bei diesen im weitesten Sinne zylinderförmigen Beruhigungskammern tritt der Flüssigkeitsnebel an einer der Stirnseiten in den Hohlraum der Beruhigungskammer ein und es entsteht aufgrund der Querschnittsvergrößerung beim Übergang vom Zuführanschluß zum Innenraum der Beruhigungskammer eine unkontrollierte Verwirbelung und insgesamt eine Verlangsamung der Flüssigkeitströpfchen in der Strahlströmung. An der gegenüberliegenden Stirnseite der Beruhigungskammer wird der Flüssigkeitsnebel in der Einatmungsphase des Inhalationsvorganges abgesaugt. Jedoch genügt bei derartigen Systemen in der Regel nicht ein einzelner Atemzug, um das gesamte Aerosolvolumen aus der Beruhigungskammer abzusaugen.

Damit für den Inhalationsvorgang eine ausreichende Menge des Flüssigkeitsnebels bereitgehalten wird, muß der Hohlraum der Beruhigungskammer relativ groß ausgelegt werden, so daß bei der unkontrollierten Verwirbelung im zur Verfügung stehenden Volumen Toträume entstehen können, in denen der Flüssigkeitsnebel vollständig zur Ruhe kommt. Diese austauscharmen Gebiete werden bei der Zerstäubung nur mit großer zeitlicher Verzögerung mit frischem Aerosol gefüllt und bei der Inhalation nur schwer wieder entleert. Zum vollständigen Entleeren sind etwa 3 bis 5 Atemzüge erforderlich. Aufgrund der langen Verweildauer in diesen Gebieten können sich die Tröpfchen an der Wandung der Beruhigungskammer niederschlagen. Daher ist die Beruhigung und Homogenisierung des Flüssigkeitsnebels in derartigen Systemen unzureichend. Ein weiterer Nachteil liegt in dem erforderlichen großen Volumen, das für den Patienten, insbesondere bei transportablen Inhalationsgeräten, nur schwer handhabbar ist.

Aus dem Deutschen Gebrauchsmuster G 89 08 273 ist ein Inhalationsgefäß aus Kunststoff bekannt, das zur Verringerung der Abmessungen eine spezielle Form besitzt. Der längliche Hauptkörper der Beruhigungskammer, der an seinem einen Ende einen Anschlußstutzen für die Zuführung des Flüssigkeitsnebels und an seinem anderen Ende einen Entnahmestutzen aufweist, besitzt insgesamt eine amphorenähnliche Form, in dem sich an den Anschlußstutzen ein kurzer Übergangsbereich anschließt, der den ellipsenähnlichen Querschnitt des Anschlußstutzens in eine Kreisform übergehen läßt und sich von da an die Form des Hauptkörpers aus kontinuierlich größer werdenden Kreisquerschnitten zusammensetzt, bis sie nach Erreichen eines größten Durchmessers sich wieder durch konstant abnehmende Kreisquerschnitte an die Größe des Entnahmestutzens annähert. Der Querschnitt dieser bekannten Beruhigungskammer nimmt also in Richtung des Aerosolstroms beginnend mit dem Durchmesser des Anschlußstutzens kontinuierlich zu und kurz vor Erreichen des Entnahmestutzens bis auf dessen Querschnitt hin ab. Auch bei diesem bekannten Inhalationsgefäß aus Kunststoff handelt es sich letztlich aber um ein sehr großes Gebilde, bei dem eine Homogenisierung und Verlangsamung des Flüssigkeitsnebels unter gleichzeitiger Bereitstellung eines ausreichenden Volumens nur unter Einschränkungen im Hinblick auf die Gesamtgröße und damit die Handhabbarkeit zugunsten des Funktionszwecks erreicht wird.

Ferner ist ein Inhalationsgefäß bekannt, das einen zylindrischen Verneblungsraum besitzt, an dessen einer Stirnseite im Mittelpunkt eine Zerstäubungsdüse vorgesehen ist. Die gegenüberliegende Stirnseite des zylindrischen Raums weist zentral ein T-Stück als Anschlußelement für den Entnahmestutzen auf, über den die Einatmung erfolgt. Um die Luftströmung im zylindrischen Raum im Hinblick auf eine Homogenisierung und Beruhigung des Flüssigkeitsnebels zu beeinflussen, ist eine den zylindrischen Raum nahezu vollständig nach oben hin verschließende Karlotte vorgesehen, die im zentralen Bereich eine für das T-Stück vorgesehene Öffnung besitzt. Im Randbereich der Kalotte wird von oben einströmende Luft an den Aussenwänden des zylindrischen Hohlkörpers entlanggeleitet und dadurch eine Durchmischung der Luft mit dem von unten eintretenden Flüssigkeitsnebel erreicht.

Der Erfindung liegt davon ausgehend die Aufgabe zugrunde, einen Vernebler insbesondere zur Anwendung in Geräten für die Inhalationstherapie zu schaffen, der bei geringen Abmessungen sowohl eine Beruhigung, als auch eine Homogenisierung des zugeführten Flüssigkeitsnebels bewirkt, und ein für den Inhalationsvorgang ausreichendes Volumen bereitstellt.

Gelöst wird diese Aufgabe durch einen Vernebler mit den Merkmalen gemäß Anspruch 1.

Weitere Ausgestaltungen der Erfindung ergeben sich aus den Unteransprüchen.

Aus WO 90/15635 ist eine Vorrichtung zur feinen Pulverisierung der Partikel eines bereits pulverförmig vorliegenden Medikaments. Der Grundkörper ist im wesentlichen zylinderförmig ausgestaltet und besitzt im Bereich beider Stirnflächen jeweils einen Anschlußstutzen, die seitlich versetzt zur Längsachse des Grundkörpers angeordnet sind. Durch den einen Anschlußstutzen wird das Medikament in einer Luftströmung zugeführt, durch den anderen zur Inhalation abgeführt; in dem Zylinder stellt sich eine spiralförmige Luftströmung ein, die derart starke Turbulenzen und Zentrifugalkräfte hervorruft, daß die Partikel des Medikaments aufeinander und auf die Innenwand des Zylinders aufprallen und weiter pulverisiert werden. Die Heftigkeit der Strömung und Teilchenbewegung und die Tatsache, daß angestrebt wird, die Medikamentpartikel gegen die Innenwand des Hohlraumes prallen zu lassen, verdeutlicht das im Vergleich zur Erfindung grundsätzlich andere Prinzip, das bei der bekannten Vorrichtung verwirklicht ist.

Die Erfindung wird im folgenden anhand von Ausführungsbeispielen und unter Bezugnahme auf die Figur genauer beschrieben, die den Aufbau eines Ausführungsbeispiels eines erfindungsgemäßen Verneblers zeigt.

Der dargestellte Vernebler gemäß der Erfindung weist einen zylinderförmigen Verneblungsraum 1 auf, der eine Öffnung 2 für die Zuführung eines Flüssigkeitsnebels und eine Öffnung 3 für die Entnahme des Flüssigkeitsnebels besitzt. Die beiden Öffnungen 2 und 3 sind in der Mantelfläche 4 des zylinderförmigen Verneblungsraumes 1 derart angebracht, daß die an den Öffnungen 2 und 3 vorgesehenen Anschlußstutzen 2' bzw. 3' tangential in die Mantelfläche 4 einmünden. Die Öffnungen 2 und 3 liegen aus diesem Grund seitlich zur Längsachse 5 versetzt, so daß sich bei Zuführung des Flüssigkeitsnebels durch die Öffnung 2 und Entnahme durch die Öffnung 3 ein um die Längsachse verlaufender Spiralwirbel ausbildet, der zu einer Füllung und Durchspülung des Verneblungsraumes 1 führt. Durch den Spiralwirbel wird dabei eine gleichmäßige Verteilung und eine Verlangsamung der Flüssigkeitströpfchen und damit eine Homogenisierung des Flüssigkeitsnebels erreicht.

Bei diesem Ausführungsbeispiel wird durch die Anordnung der beiden Öffnungen 2 und 3 die Ausbildung des Spiralwirbels gewährleistet, da die Zuführung und die Absaugung des Wirkstoffaerosols aus dem zylinderförmigen Verneblungsraum tangential erfolgen. Die seitlich zur Längsachse 5 versetzte Öffnung 3 ist aus diesem Grund so angeordnet, daß der sich nach der Zuführung des Flüssigkeitsnebels ausbildende Spiralwirbel praktisch in die Öffnung 3 einmündet. Jedoch sind die Flüssigkeitströpfchen auf dem durch den Spiralwirbel verlängerten Weg durch den Verneblungsraum soweit verlangsamt worden, daß ein unerwünscht starker Aerosolstrahl an der Öffnung 3 vermieden wird.

Die Lage der Öffnungen 2 und 3 in unmittelbarer Nähe der Stirnflächen 6 und 7 des zylinderförmigen Verneblungsraumes 1 gewährleistet eine optimale Ausnutzung des zur Verfügung stehenden Volumens des Verneblungsraumes 1 und eine möglichst große Weglänge mit gleicher Verweildauer für die Flüssigkeitströpfchen.

Wird in einen entleerten Verneblungsraum 1 ein Wirkstoffaerosol über den Anschlußstutzen 2' und die Öffnung 2 zugeführt, so füllt sich der Verneblungsraum 1 unter Ausbildung eines Spiralwirbels mit kleiner Ganghöhe, so daß die eintretenden Flüssigkeitströpfchen vom Bereich der Öffnung 2 her beginnend den Verneblungsraum 1 ausfüllen. Für die Flüssigkeitströpfchen ergibt sich aufgrund der geringen Ganghöhe ein sehr langer Weg, der einem Vielfachen der Länge des zylinderförmigen Verneblungsraumes 1 entspricht. Beim Füllvorgang bildet sich somit ein homogener Flüssigkeitsnebel aus schwebenden Flüssigkeitströpfchen innerhalb des Verneblungsraumes 1, der in der Einatmungsphase des Inhalationsvorganges durch die Öffnung 3 und über den Anschlußstutzen 3' mit einem einzigen Atemzug vollständig abgesaugt wird. Dabei kann aber kontinuierlich weiterhin durch die Öffnung 2 das Wirkstoffaerosol zugeführt werden.

Während des Füllvorgangs wird der Verneblungsraum 1 nur mit dem für die Zerstäubung erforderlichen Luftvolumenstrom durchströmt, so daß ein Aerosol mit hoher Tröpfchenkonzentration entsteht. Bei der Einatmung erhöht sich der durchgesetzte Volumenstrom entsprechend dem Atemzug des Patienten, so daß das nachströmende Aerosol eine deutlich geringere Konzentration aufweist. Der sich nun einstellende Spiralwirbel weist eine größere Ganghöhe auf, da die Strömung der Flüssigkeitströpfchen im Verneblungsraum 1 durch den Absaugvorgang über die Öffnung 3 beschleunigt wird. Dennoch wird eine ausreichende Verlangsamung der Flüssigkeitströpfchen erreicht, da die Weglänge aufgrund des Spiralwirbels weiterhin sehr viel größer als die einfache Länge des zylinderförmigen Verneblungsraumes 1 ist. Auch die Homogenisierung des Flüssigkeitsnebels ist bei der auch während des Absaugvorgangs erfolgenden Ausbildung des Spiralwirbels gewährleistet.

In der Ausatmungsphase wird ein Zurückströmen der Atemluft in den Verneblungsraum 1 auf bekannte Weise, etwa durch ein Rückschlagventil, verhindert. Dadurch wird eine Behinderung der Ausbildung des Spiralwirbels vermieden, so daß in der Ausatmungsphase des Inhalationsvorganges erneut die Füllung des Verneblungsraumes 1 mit dem durch die Öffnung 2 zugeführten Flüssigkeitsnebel unter Ausbildung eines Spiralwirbels mit kleiner Ganghöhe bei hoher Tröpfchenkonzentration einsetzt.

Das Volumen des Verneblungsraumes 1 muß derart ausgelegt sein, daß einerseits ein Großteil des Atemzugvolumens des Patienten abgedeckt ist, und andererseits möglichst der gesamte Verneblungsraum während der Ausatmungsphase wieder mit hoch konzentriertem Aerosol gefüllt werden kann. Dabei wird das während der Einatmung entstandene Aerosol mit niedriger Teilchenkonzentration aus dem Verneblungsraum verdrängt. Der daraus resultierende Wirkstoffverlust ist jedoch im Vergleich zu herkömmlichen kontinuierlich betriebenen Verneblersystemen gering.

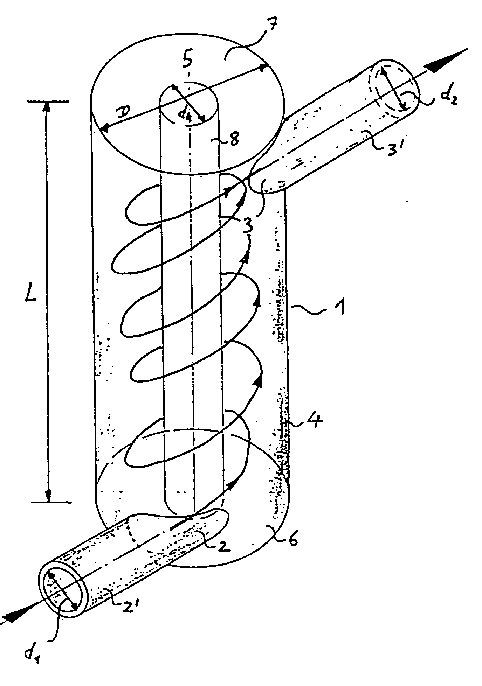

Als besonders geeignet für den praktischen Einsatz wurden unter den zuvor erwähnten Gesichtspunkten Vernebler gefunden, die ein Volumen von 200 bis 350 ml (Erwachsener) bzw. 100 bis 200 ml (Kind) besitzen. Die in Fig. 1 angedeuteten Maße L und D sind vorteilhaft in einem Verhältnis L/D von ca. 2 bis 3 zu wählen. Vorzugsweise beträgt das Volumen 300 ml (Erwachsener) bzw. 150 ml (Kind) und das Verhältnis L/D ca. 2,5.

Die Ausbildung des Spiralwirbels wird weiter unterstützt durch die Anordnung eines ebenfalls zylinderförmigen Kernteils 8 in der Mitte des Verneblungsraumes 1, das sich in Längsrichtung erstreckt und dessen Längsachse mit der Längsachse 5 des Verneblungsraumes 1 zusammenfällt. Das Kernteil 8 kann dabei, wie in der Figur gezeigt, einen konstanten Querschnitt oder aber einen sich ändernden und die Geschwindigkeit der Flüssigkeitströpfchen beeinflussenden Querschnitt besitzen. Wesentlich ist dabei die die Ausbildung des Spiralwirbels unterstützende Funktion des Kernteils 8, die zu einer Vielzahl von Gestaltungsmöglichkeiten führen kann. Wichtiges Gestaltungskriterium ist z.B. ein schnelles und vollständiges Absaugen des konzentrierten Aerosols in der Einatmungsphase, mit dem Ziel, die Verweildauer der einströmenden Flüssigkeitströpfchen weitgehend konstant zu halten und eine optimale Ausnutzung des für die Aufnahme des Flüssigkeitsnebels zur Verfügung stehenden Speichervolumens des Verneblungsraumes 1 sicherzustellen.

Der Durchmesser $d_k$ des Kernteils wird vorteilhaft so gewählt, daß das Verhältnis $D/d_k$ etwa 2,5 bis 3,5, vorzugsweise 3,0 beträgt. Die Durchmesser $d_1$ und $d_2$ der Anschlußstutzen 2' bzw. 3' erfüllen vorteilhaft die Bedingungen $d_1/d_k = 0,5$ bis $1,5$ und $d_2/d_k = 0,5$ bis $1,5$, vorzugsweise die Bedingung $d_1 = d_2 = d_k$.

**Patentansprüche**

1. Vernebler zur Anwendung in Geräten für die Inhalationstherapie

   -- mit einem Verneblungsraum (1), der im wesentlichen zylinderförmig ausgestaltet ist, dessen Größe an das Atemzugvolumen eines Patienten angepaßt ist und der eine Beruhigung des Aerosols bewirkt,

   -- mit einer ersten Anschlußöffnung (2) im Bereich der einen Stirnfläche (6) des Verneblungsraumes (1) für die Zuführung eines Flüssigkeitsnebels, die in der Mantelfläche (4) des Verneblungsraumes (1) seitlich versetzt zur Längsachse (5) des Verneblungsraumes (1) angeordnet ist,

   -- mit einer zweiten Anschlußöffnung (3) für die Entnahme des Flüssigkeitsnebels, die in der Mantelfläche (4) des Verneblungsraumes (1) und in der Nähe der anderen Stirnfläche (7) des Verneblungsraumes (1) seitlich versetzt zur Längsachse (5) des Verneblungsraumes (1) angeordnet ist, und

   -- mit einem zylinderförmigen Kernteil (8), das im Verneblungsraum (1) vorgesehen ist, das sich in Längsrichtung des Verneblungsraumes erstreckt und dessen Längsachse mit der Längsachse (5) des Verneblungsraumes (1) zusammenfällt.

2. Vernebler nach Anspruch 1,
   dadurch **gekennzeichnet**, daß
   der Verneblungsraum (1) ein Volumen von 100 bis 350 ml, vorzugsweise 150 bis 300 ml besitzt.

3. Vernebler nach einem der Ansprüche 1 oder 2,
   dadurch **gekennzeichnet**, daß
   die Länge L des Verneblungsraumes (1) zum Durchmesser D des Verneblungsraumes (1) in einem Verhältnis L/D von ca. 2 bis 3, vorzugsweise 2,5 steht.

4. Vernebler nach einem der Ansprüche 1 bis 3,
   dadurch **gekennzeichnet**, daß
   der Durchmesser $d_k$ des Kernteils (8) zum Durchmesser D des Verneblungsraumes (1) in einem Verhältnis $D/d_k$ von ca. 2,5 bis 3,5, vorzugsweise 3,0 steht.

5. Vernebler nach einem der vorhergehenden Ansprüche,
   dadurch **gekennzeichnet**, daß
   zylindrische Anschlußstutzten (2', 3') an den Öffnungen (2, 3) angeordnet sind.

6. Vernebler nach Anspruch 5,
   dadurch **gekennzeichnet,** daß
   die Anschlußstutzen (2', 3') Durchmesser $d_1$ und $d_2$ im Verhältnis $d_1/d_k$ bzw. $d_2/d_k$ von 0,5 bis 1,5, vorzugsweise 1 stehen.

**Claims**

1. Nebuliser for use in appliances for inhalation therapy

   - with a nebulising chamber (1) which is essentially of cylindrical shape, of which the size is adapted to the breath volume of a patient and which causes stabilisation of the aerosol,

- with a first connecting opening (2) in the region of one end face (6) of the nebulising chamber (1) for the supply of a liquid mist, which is arranged in the peripheral surface (4) of the nebulising chamber (1), laterally offset from the longitudinal axis (5) of the nebulising chamber (1),

- with a second connecting opening (3) for extraction of the liquid mist, which is arranged in the peripheral surface (4) of the nebulising chamber (1) and in the vicinity of the other end face (7) of the nebulising chamber (1), laterally offset from the longitudinal axis (5) of the nebulising chamber (1), and

- with a cylindrical core portion (8) which is provided in the nebulising chamber (1), which extends in the longitudinal direction of the nebulising chamber and of which the longitudinal axis coincides with the longitudinal axis (5) of the nebulising chamber (1).

2. Nebuliser according to claim 1, characterised in that the nebulising chamber (1) has a volume of 100 to 350 ml, preferably 150 to 300 ml.

3. Nebuliser according to either of claims 1 or 2, characterised in that the length L of the nebulising chamber (1) relative to the diameter D of the nebulising chamber (1) is in a ratio L/D of about 2 to 3, preferably 2.5.

4. Nebuliser according to any of claims 1 to 3, characterised in that the diameter $d_k$ of the core portion (8) relative to the diameter D of the nebulising chamber (1) is in a ratio $D/d_k$ of about 2.5 to 3.5, preferably 3.0.

5. Nebuliser according to any of the preceding claims, characterised in that cylindrical connecting pieces (2', 3') are arranged at the openings (2, 3).

6. Nebuliser according to claim 5, characterised in that the connecting pieces (2', 3') have diameters $d_1$ and $d_2$ in a ratio $d_1/d_k$ and $d_2/d_k$ of 0.5 to 1.5, preferably 1.

## Revendications

1. Nébuliseur pour utilisation dans des appareils pour thérapie par inhalation,

comprenant une chambre de nébulisation (1) de configuration essentiellement cylindrique, dont la taille est adaptée au volume d'inhalation d'un patient et qui réalise une stabilisation de l'aérosol,

comprenant, dans la région d'une des surfaces frontales (6) de la chambre de nébulisation (1), une première ouverture de raccordement (2) pour l'amenée d'un nuage de liquide, qui est aménagée dans la surface d'enveloppe (4) de la chambre de nébulisation (1), décalée latéralement par rapport à l'axe longitudinal (5) de la chambre de nébulisation (1),

comprenant une seconde ouverture de raccordement (3) pour la reprise du nuage de liquide, qui est aménagée dans la surface d'enveloppe (4) de la chambre de nébulisation (1) et au voisinage de l'autre surface frontale (7) de la chambre de nébulisation (1), décalée latéralement par rapport à l'axe longitudinal (5) de la chambre de nébulisation (1),

comprenant un noyau cylindrique (8), prévu dans la chambre de nébulisation (1), qui s'étend dans la direction longitudinale de la chambre de nébulisation et dont l'axe longitudinal coïncide avec l'axe longitudinal (5) de la chambre de nébulisation (1).

2. Nébuliseur selon la revendication 1, caractérisé en ce que la chambre de nébulisation (1) possède un volume de 100 à 350 ml, de préférence de 150 à 300 ml.

3. Nébuliseur selon l'une des revendications 1 ou 2, caractérisé en ce que la longueur L de la chambre de nébulisation (1) est par rapport au diamètre D de la chambre de nébulisation (1) dans un rapport L/D d'environ 2 à 3, de préférence de 2,5.

4. Nébuliseur selon l'une des revendications 1 à 3, caractérisé en ce que le diamètre $d_k$ du noyau (8) est par rapport au diamètre D de la chambre de nébulisation (1) dans un rapport $D/d_k$ d'environ 2,5 à 3,5, de préférence de 3,0.

5. Nébuliseur selon l une des revendications précédentes, caractérise en ce que des raccords cylindriques (2', 3') sont disposés sur les ouvertures (2, 3).

6. Nébuliseur selon la revendication 5, caractérisé en ce que les diamètres $d_1$ et $d_2$ des raccords (2', 3') présentent des rapports respectifs $d_1/d_k$ et $d_2/d_k$ de 0,5 à 1,5, de préférence de 1.